# EUROPEAN PATENT SPECIFICATION

(11) **EP 2 522 327 B1**
(45) Date of publication and mention of the grant of the patent: **03.06.2020**
(21) Application number: 10842192.6
(22) Date of filing: 20.12.2010
(51) Int. Cl.: A61K 8/29, A61K 8/81, A61Q 3/02

(54) **AQUEOUS COSMETIC PREPARATION**
WÄSSRIGES KOSMETIKPRÄPARAT
PRÉPARATION COSMÉTIQUE AQUEUSE

(30) Priority: 07.01.2010 JP 2010001868; 06.01.2010 JP 2010001041
(43) Date of publication of application: 14.11.2012
(73) Proprietor: Mitsubishi Pencil Company, Limited, Tokyo 140-8537 (JP)
(72) Inventor: SAKUMA Satoshi, Fujioka-shi Gunma 375-8501 (JP); HAYAKAWA Takayuki, Fujioka-shi Gunma 375-8501 (JP)
(74) Representative: Vossius & Partner Patentanwälte Rechtsanwälte mbB
(86) International application number: PCT/JP2010/072913
(87) International publication number: WO 2011/083679

(56) References cited:
- EP-A1- 0 980 900
- EP-A1- 2 008 640
- WO-A1-98/26011
- JP-A- 6 211 631
- JP-A- 8 157 329
- JP-A- 9 071 511
- JP-A- 9 110 642
- JP-A- 10 265 338
- JP-A- 10 265 339
- JP-A- 11 158 039
- JP-A- 2002 114 641
- JP-A- 2003 171 220
- JP-A- 2003 171 234
- JP-A- 2004 059 035
- JP-A- 2007 077 115
- DATABASE GNPD [Online] MINTEL; 29 May 2008 (2008-05-29), "Calcium Power Gloss Nail Lacquer", XP002739670, Database accession no. 910007

## Description

### Technical Field

The present invention relates to an aqueous cosmetic containing titanium oxide and an acid dye or an organic pigment, more specifically to an aqueous cosmetic which is excellent in an aging settling stability of titanium oxide to make a hard cake less liable to be produced even when titanium oxide settles down and can readily be redispersed by stirring and which is excellent in usability and a coating performance and suited to a manicure composition for a nail art, cosmetics for skin coloring and the like.

### Background Art

Many cosmetics have so far been known as aqueous cosmetics containing titanium oxide which is excellent in a masking power.

Titanium oxide contained in the above aqueous cosmetics settles down when left standing still for long time to form a hard cake. This hard cake involves the problem that it is not readily redispersed by stirring and the like.

Cosmetics for skin coloring comprising at least water, a pigment containing titanium oxide and a resin have so far been known as cosmetics for skin coloring which have good redispersibility of a pigment in storing, wherein contained are at least one rein selected from the group consisting of a styrene-acrylic acid copolymer, a styrene-maleic acid copolymer, a styrene-alkyl acrylate copolymer and a styrene-alkyl maleate copolymer and a base such as ammonia and amines, and at least one thickener selected from a clay base inorganic thickener, an inorganic fine particle thickener and a polysaccharide thickener is contained (refer to, for example, patent document 1 filed by the present applicants).

Further, known is an aqueous manicure composition in which dispersibility and the like are improved by blending a powder obtained by subjecting a powder of titanium oxide and the like to hydrophilic treatment with a silylating agent having a polyoxyethylene chain and an acryl base polymer emulsion (refer to, for example, patent document 2).

However, also in aqueous cosmetics such as the aqueous cosmetics for skin coloring and the aqueous manicure composition each described in the above patent documents 1 and 2, the existing state is that the components settle down in a certain case when left standing still for long time to form hard cakes and that it is not easy to redisperse them by stirring and the like.

Also, involved therein are the problems that when an acid dye is present in an aqueous cosmetic containing titanium oxide, aggregation of titanium oxide is liable to be brought about and that when sodium-polyaspartate is used as a dispersant for titanium oxide, aggregation thereof is brought about with the passage of time to cause a rise in the viscosity. Accordingly, the problem that they cannot be loaded and used in an applicator of a pen type is involved therein.

Further, when an aqueous cosmetic containing titanium oxide is loaded in an applicator of a pen type, a pen tip thereof is dried, and therefore involved therein is the problem that particularly in a case of an aqueous cosmetic in which titanium oxide is blended with an organic pigment, the above organic pigment is aggregated and solidified, whereby the cakes become harder.

Patent document 3 discloses a cosmetic for skin coloring which contains at least water, a pigment and a resin, at least one kind selected from a styrene-acrylic acid copolymer, a styrene-maleic acid copolymer, a styrene-alkyl acrylate copolymer and a styrene-alkyl maleate copolymer as the resin and at least a base selected from ammonia, amines and alkali (earth) metal hydroxide.

Patent document 4 discloses a water based liquid makeup cosmetic which is filled in an applicator for a liquid cosmetic equipped with a brush-like coating part to be suitably used for making up, particularly a water based liquid makeup cosmetic which is suited for making up around eyes, and in order to obtain the cosmetic, assumed is a formation comprising at least a tabular pigment, a pigment dispersant, a coating film-forming agent, 0.001 to 0.5 mass % of a surfactant and water and further comprising a spherical powder.

Patent document 1: Japanese Patent Application Laid-Open No. 157329/1996 (claims, examples and others)
Patent document 2: Japanese Patent Application Laid-Open No. 110642/1997 (claims, examples and others)
Patent document 3: JP-H08-157329 A
Patent document 4: EP 2 008 640 A1

### DISCLOSURE OF THE INVENTION

In light of the problems on the conventional arts described above, that is, a problem of an aqueous cosmetic containing titanium oxide and an acid dye and a problem of an aqueous cosmetic containing titanium oxide and an organic pigment, the present invention intends to solve them, and an object thereof is to provide an aqueous cosmetic containing titanium oxide and an acid dye or an organic pigment, wherein it is excellent in an aging settling stability to make a hard cake less liable to be produced even when titanium oxide settles down and can readily be redispersed by stirring, and it inhibits a viscosity from rising and is excellent in usability and a coating performance.

In light of the problems on the conventional arts described above, the present inventors intend to solve them, and they have found that the respective aqueous cosmetics meeting the objects described above are obtained by adding at least a specific amount of titanium oxide, an acid dye or an organic pigment, an acryl resin having specific physical properties and water. Thus, the present invention has come to be completed.

That is, the present invention comprises in the following items (1) to (4).
(1) An aqueous cosmetic containing at least 5 to 30 % by mass of titanium oxide, an acid dye, an alkali-soluble type acryl resin and water or
   containing at least 5 to 30 % by mass of titanium oxide, an organic pigment, an alkali-soluble type acryl resin and water, wherein the alkali-soluble type acryl resin is an (alkyl acrylate/ octylacrylamide) copolymer, and wherein a mass ratio of the alkali-soluble type acryl resin to titanium oxide is 0.04 to 1, based on 1 of titanium oxide.
(2) The aqueous cosmetic as described in the above item (1), wherein titanium oxide coated on a surface with alumina is contained.
(3) The aqueous cosmetic as described in the above item (1) or (2), wherein in measuring a viscosity of the aqueous cosmetic at 25°C by means of a cone plate type viscometer, a viscosity thereof at a shear rate of 3.83 s⁻¹ is 150 mPa·s or less, and a viscosity thereof at a shear rate of 383 s⁻¹ is 20 mPa·s or less.
(4) The aqueous cosmetic as described in any one of the above items (1) to (3), wherein the aqueous cosmetic is loaded in a liquid storing part of an applicator which is equipped with at least the liquid storing part and an applying member and in which a liquid is transported from the liquid storing part to the applying member by virtue of a capillary force.

### Effects of the invention

According to the present invention, provided are an aqueous cosmetic containing titanium oxide and an acid dye, or an aqueous cosmetic containing titanium oxide and an organic pigment, as specified in appended claim 1, wherein both of them are excellent in an aging settling stability to make a hard cake less liable to be produced even when titanium oxide settles down and can readily be redispersed by stirring, and they inhibit a viscosity from rising with the passage of time and are excellent in usability and a coating performance and suited to a manicure composition for a nail art, cosmetics for skin coloring and the like.

### BRIEF DESCRIPTION of THE DRAWINGS

Fig. 1 is a partial vertical cross section and a left side face drawing which show one example of an applicator loading the aqueous cosmetic of the present invention.
Fig. 2 (a) is a side face drawing showing one example of the applicator, and (b) is a I-I line cross section of (a).
Fig. 3 is a partial vertical cross section and a left side face drawing which show another example of an applicator loading the aqueous cosmetic of the present invention.

### MODE FOR CARRYING OUT THE INVENTION

The embodiments of the present invention shall be explained below in detail.

The aqueous cosmetic of the present invention is characterized by that it contains 5 to 30 % by mass of titanium oxide, an acid dye, an alkali-soluble type acryl resin and water, or that it contains 5 to 30 % by mass of titanium oxide, an organic pigment, an alkali-soluble type acryl resin and water, wherein the alkali-soluble type acryl resin is an (alkyl acrylate/octylacrylamide) copolymer and wherein a mass ratio of the alkali-soluble type acryl resin to titanium oxide is 0.04 to 1, based on 1 of titanium oxide.

Titanium oxide used in the present invention exerts an excellent masking power when coated on a skin and a nail, and it shall not specifically be restricted as long as it is used usually for aqueous cosmetics. Titanium oxide subjected to alumina treatment is preferably used in terms of controlling a surface activity.

For example, at least one (each alone or a mixture of two or more kinds, hereinafter the same shall apply) of A-100 and W-100 (all manufactured by Ishihara Sangyo Kaisha, Ltd.), JR and JA-1 (all manufactured by Tayca Corporation) and the like which are commercially available can be used as titanium oxide which can be used, and at least one of CR50, CR60, CR67, PF737 and R680 (all manufactured by Ishihara Sangyo Kaisha, Ltd.), R900 (manufactured by Du Pont Co., Ltd.) and the like which are commercially available can be used as titanium oxide subjected to alumina treatment.

A content of above titanium oxide is 5 to 30 % by mass (hereinafter referred to merely as "%"), preferably 8 to 25 % based on the total amount of the aqueous cosmetic.

If a content of above titanium oxide is less than 5 %, the coating film is poor in a masking property. On the other hand, if it exceeds 30 %, a viscosity of the aqueous cosmetic is instable, and both are not preferred.

The acid dye used in the present invention is used as a color material and shall not specifically be restricted as long as it is a dye used usually as an acid dye for aqueous cosmetics.

For example, dyes which have so far been used for cosmetics and the like and which have high safety to human bodies are preferred as the above acid dye, and they include, for example, nitro dyes, azo, dyes, nitroso dyes, triphenylmethane dyes, xanthene dyes, quinoline dyes, anthraquinone dyes, indigo dyes and the like. To be specific, capable of being preferably listed from the viewpoint of a dyeing power are Red No. 2, Red No. 3 (FD & C Red No. 3), Red No. 40 (FD & C Red No. 40), Red No. 102, Red No. 104 (D & C Red No. 28), Red No. 105, Red No. 106, Red No. 201 (D & C Red No. 6), Red No. 202 (D & C Red No. 7), Red No. 203, Red No. 205, Red No. 227 (D & C Red No. 33), Red No. 230-1 (D & C Red No. 22), Red No. 401, Red No. 402, Red No. 504 (FD & C Red No. 4), Orange No. 205 (D & C Orange No. 4), Orange No. 402, Yellow No. 4 (FD & C Yellow No. 5), Yellow No. 5 (FD & C Yellow No. 6), Yellow No. 203 (D & C Yellow No. 10), Yellow No. 402, Yellow No. 403-1 (Ext. D & C Yellow No. 7), Yellow No. 406, Yellow No. 407, Green No. 3 (FD & C Green No. 3), Green No. 201, Green No. 402, Blue No. 1 (FD & C Blue No. 1), Blue No. 2 (FD & C Blue No. 2), Blue No. 203, Blue No. 205 (D & C Blue No. 4), Blue No. 403, Blue No. 404, Brown No. 201 (D & C Brown No. 1), Violet No. 401 (Ext. D & C Violet No. 2), Black No. 401 and the like. At least one selected from the above acid dyes can be used and shall not specifically be restricted as long as it is an acid dye used for aqueous cosmetics.

A content of the above acid dyes is 0.02 to 20 %, preferably 0.2 to 10 % based on the total amount of the aqueous cosmetic in terms of a color developing property, a suitable viscosity and a smooth discharge property in an aqueous cosmetic applicator equipped with an applying member.

If a content of the above acid dye is less than 0.02 %, the good developed color is not obtained. On the other hand, if it exceeds 20 %, the cosmetic is increased in a viscosity and liable to be reduced in a discharge property and bring about aggregation, and therefore both are not preferred.

Next, the organic pigment used in the present invention is used as a color material and shall not specifically be restricted as long as it is a pigment used usually as an organic pigment for aqueous cosmetics.

It includes, for example, at least one selected from organic pigments such as Blue No. 1 Al Lake, Red No. 202, Red No. 220, Red No. 226, Red No. 228, Blue No. 201, Blue No. 204, Blue No. 404, Yellow No. 401, Yellow No. 205, Yellow No. 4 Al Lake, Yellow No. 203 Al Lake, and Red No. 104 Al Lake. It shall not specifically be restricted as long as it is an organic pigment used for aqueous cosmetics.

A content of the above organic pigments is preferably 0.05 to 15 %, more preferably 0.1 to 10 % based on the total amount of the aqueous cosmetic in terms of a color developing property, a suitable viscosity and a smooth discharge property in an aqueous cosmetic applicator equipped with an applying member.

If a content of the above organic pigments is less than 0.05 %, the good developed color is not obtained. On the other hand, if it exceeds 15 %, the cosmetic is increased in a viscosity and liable to be reduced in a discharge property and bring about aggregation, and therefore both are not preferred.

The alkali-soluble type acryl resin used in the present invention functions as a dispersant for titanium oxide and the like in the aqueous cosmetic and functions as a fixing resin after coated. It includes, to be specific, at least one of (alkyl acrylate/octylacrylamide) copolymers, alkyl acrylate copolymers and the like, and to be more specific, it has a carboxyl group in a structure and is soluble at an alkaline range.

In the present invention, the composition is suitably controlled by a pH controller and the like until the alkali-soluble type acryl resin is dissolved. Capable of being used as the pH controller are 2-amino-2-methyl-1-propanol, 2-amino-2-methyl-1,3-propanediol, triethanolamine, L-arginine, aqueous ammonia, sodium hydroxide and the like, and 2-amino-2-methyl-1-propanol is particularly preferred. Also, when the alkali-soluble type acryl resin described above is once dissolved at an alkaline range, it has stable solubility up to the vicinity of pH 7.0.

Commercially available products such as AMPHOMER V-42 (manufactured by Akzo Nobel Corporate) which is an (alkyl acrylate/octylacrylamide) copolymer, Luvimer 100P (manufactured by BASF A.G.) which is an alkyl acrylate copolymer and DERMACRYL AGF (manufactured by Akzo Nobel Corporate) which is an alkyl acrylate copolymer emulsion can be used as the alkali-soluble type acryl resin.

In the present invention, the (alkyl acrylate/octylacrylamide) copolymer is
used in terms of further improving dispersibility of titanium oxide.

A content of the above alkali-soluble type acryl resins is preferably 0.05 to 30 %, more preferably 0.1 to 20 % in terms of a solid content based on the total amount of the aqueous cosmetic from the viewpoints of dispersion stability and a fixing property of titanium oxide.

If a content of the above alkali-soluble type acryl resin is less than 0.05 %, the sufficiently high pigment dispersion ability is not obtained. On the other hand, if it exceeds 30 %, an influence comes to be exerted on the aging stability, and therefore both are not preferred.

In the present invention, the alkali-soluble type acryl resin is used as a suited dispersant for titanium oxide, and a mass ratio of the alkali-soluble type acryl resin to titanium oxide is **0.04 to 1, preferably**
0.04 to 0.8 based on 1 of titanium oxide.

If the above mass ratio is less than 0.01, further effects of the present invention can not be exerted, and even if it exceeds 1, no problems are raised as long as it does not exceed an upper limit of a content of the alkali-soluble type acryl resin.

In the present invention, a water-soluble organic solvent is preferably contained in terms of improving the finishing property and a moisturizing property of an applying member such as a pen tip of the applicator.

The water-soluble organic solvent which can be used includes water-soluble glycols such as 1,3-bytylene glycol, 1,4-bytylene glycol, pentylene glycol, ethylene glycol, diethylene glycol, polyethylene glycol, propylene glycol, dipropylene glycol, and glycerin.

A content of the above water-soluble organic solvents is 1 to 20 %, preferably 8 to 15 % based on the total amount of the aqueous cosmetic.

If a content of the above water-soluble organic solvent (water-soluble glycols) is less than 1 %, an effect of adding the water-soluble organic solvent can not be exerted, and on the other hand, if it exceeds 20 %, an adverse effect is exerted on suitable dispersion of titanium oxide in a certain case.

The aqueous cosmetic of the present invention can further contain ethanol and the like for a quick drying property. Still further, it can contain suited amounts of a chelating agent, a thickener (natural saccharides such as xanthan gum), a water-soluble polymer, various surfactants for reducing a surface tension, a fungicide, an antioxidant, a UV absorber, a fragrance and the like which are usually used for liquid cosmetics as long as the effects of the present invention are not damaged.

The fungicide which can be used includes parabens, sodium dehydroacetate, phenoxyethanol and the like. The fungicide used in the present invention includes antiseptic agents, and capable of being used as parabens which are antiseptic agents are methyl paraoxybenzoate, ethyl paraoxybenzoate, propyl paraoxybenzoate, butyl paraoxybenzoate, isopropyl paraoxybenzoate and the like.

In the aqueous cosmetic of the present invention, the respective components described above are contained in both of the acid dye base aqueous cosmetic and the organic pigment base aqueous cosmetic, and the balance is controlled by water (purified water, ion-exchanged water, distilled water and the like). In measuring a viscosity of the aqueous cosmetic at 25°C by means of a cone plate type viscometer, a viscosity thereof at a shear rate of 3.83 s⁻¹ is 150 mPa·s or less, and a viscosity thereof at a shear rate of 383 s⁻¹ is 20 mPa·s or less.

If the viscosity described above at a shear rate of 3.83 s⁻¹ exceeds 150 mPa·s and/or if the viscosity described above at a shear rate of 383 s⁻¹ exceeds 20 mPa·s, the followability is reduced, and both are not preferred.

In order to satisfy the viscosities at the respective shear rates described above, titanium oxide, the acid dye or the organic pigment, the alkali-soluble type acryl resin and the like which are added to the aqueous cosmetic are suitably combined in the ranges of the respective contents described above, whereby the viscosities can be controlled. Also, they can be controlled by suitably using a thickener (natural polysaccharides, mineral base thickeners and the like) and the like.

The aqueous cosmetic of the present invention is prepared by mixing and dispersing the respective components of the acid dye base aqueous cosmetic and the organic pigment base aqueous cosmetic in the contents of the respective ranges described above by means of a mixing dispersing equipment, for example, a bead mill, a homomixer, a disper, an attritor, a ball mill, a sand grinder and the like.

At least titanium oxide, the acid dye and the alkali-soluble type acryl resin are contained in the acid dye base aqueous cosmetic of the present invention thus constituted, or at least titanium oxide, the organic pigment and the alkali-soluble type acryl resin are contained in the organic pigment base aqueous cosmetic of the present invention, whereby both aqueous cosmetics are controlled so that titanium oxide is stably dispersed in water to provide the aqueous cosmetics with prescribed physical properties. Accordingly, obtained are the aqueous cosmetics which are excellent in an aging settling stability to make a hard cake less liable to be produced even when titanium oxide settles down and can readily be redispersed by lightly stirring and which inhibit a viscosity from rising with the passage of time and are excellent in usability and a coating performance and suited to a manicure composition for a nail art, cosmetics for skin coloring and the like.

Also, in the acid dye base aqueous cosmetic of the present invention, the alkali-soluble type acryl resin which is an (alkyl acrylate/octylacrylamide) copolymer is added as a dispersant for titanium oxide, whereby the excellent dispersion performance is exerted in a case in which the acid dye is present rather than in a case in which sodium polyaspartate is used as a dispersant for titanium oxide and a case in which a styrene-acryl base resin is used as a dispersant for titanium oxide. This is estimated to be attributable to that a soft cake can be formed due to the absence of a styrene group.

Further, in the acid dye base aqueous cosmetic of the present invention, titanium oxide is not aggregated and solidified and a viscosity thereof can be inhibited from rising with the passage of time, even when the aqueous cosmetic containing titanium oxide and the acid dye is loaded in an applicator of a pen type described later. Accordingly, the aqueous cosmetic which has not so far been available and which can suitably be used in an applicator of a pen type is obtained.

Also, in the organic pigment base aqueous cosmetic of the present invention, the alkali-soluble type acryl resin which is an (alkyl acrylate/octylacrylamide) copolymer is added as a dispersant for titanium oxide and the like, whereby more excellent dispersion performance than those of conventional styrene-acryl base resins and the like is exerted. This is estimated to be attributable to that a soft cake can be formed due to the absence of a styrene group.

Further, in the organic pigment base aqueous cosmetic of the present invention, even when the aqueous cosmetic containing titanium oxide is loaded in an applicator of a pen type described later, and therefore the aqueous cosmetic in which aggregation and solidification of the organic pigment due to drying of a pen tip is not caused and which has not so far been available is obtained as well particularly in the aqueous cosmetic blended with the organic pigment.

Both of the acid dye base aqueous cosmetic or the organic pigment base aqueous cosmetic of the present invention thus constituted is loaded in an applicator of a pen type, to be specific, an applicator which is equipped with at least a liquid storing part and an applying member and in which a liquid is transported from the liquid storing part to the applying member by virtue of a capillary force, wherein the aqueous cosmetic is loaded in the liquid storing part described above, and the applicator comes to be usable.

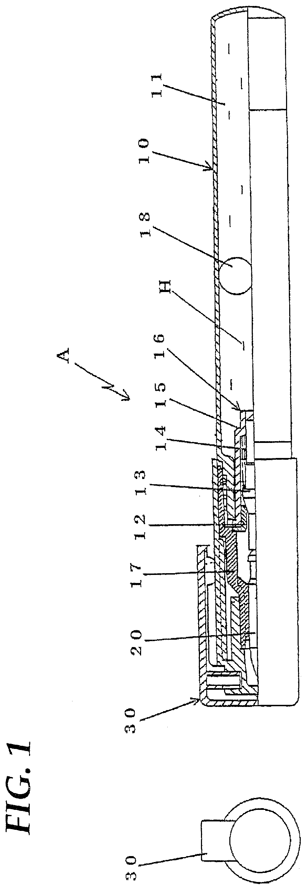

For example, an applicator A which is equipped, as shown in Fig. 1, with a liquid storing part 11 stored therein with the aqueous cosmetic (aqueous manicure composition in the present embodiment) constituted above and an applying member 20 having a capillary force and in which an acid dye base or organic pigment base aqueous cosmetic H is transported from the liquid storing part 11 to the applying member 20 by virtue of a capillary force can be used as an aqueous cosmetic-stored applicator loading the aqueous cosmetic of the present invention.

To describe the above applicator A in detail, an inside of an applicator main body 10 is the liquid storing part 11; a valve mechanism 16 comprising a valve sheet 12, a valve rod 13, a spring member 14 comprising a coil spring and a spring bearing 15 is provided in front of the applicator main body 10, and the applying member 20 described above is slidably held in an inside of a front shaft member 17 mounted in a front of the applicator main body 10. Meanwhile, reference numeral 18 shown in the drawing is a stirring ball made of SUS, and a reference numeral 30 is a cap member.

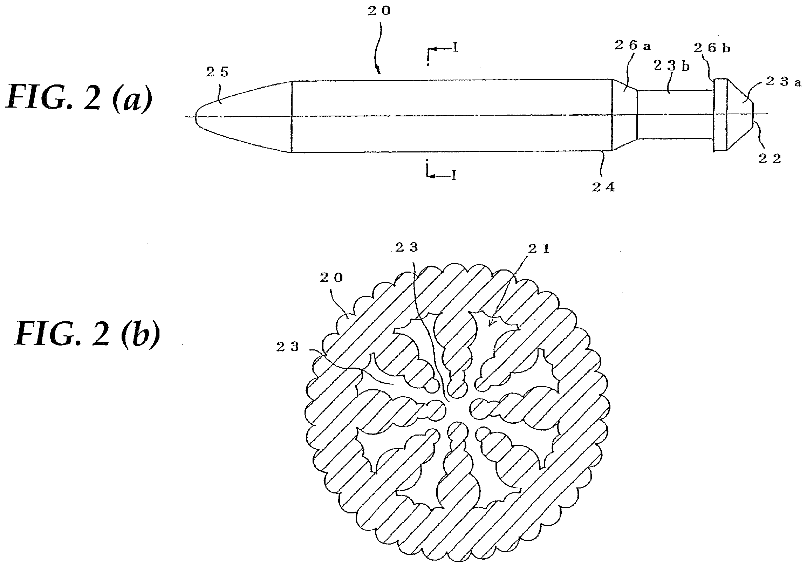

The applying member 20 provided with a capillary force is preferably, as shown in Fig. 2 (a) and (b), a member which is equipped with a tubular inner cylinder 21 and in which an applying tip 25 is equipped with a slit in a conical and radial form. To describe the applying member 20 in detail, a rear end part 22 of the applying member 20 is brought into contact with a front end of the valve rod 13, and the tubular inner cylinder 21 is continuously provided in an axial line direction with a cosmetic passage 23 of a cross section-radial inner groove through which the aqueous cosmetic passes. The above applying member 20 is constituted from a synthetic resin such as polyacetal, and holes 23a, 23b for introducing the aqueous cosmetic into the passage 23 in the applying member 20 are formed in the rear end part 22 and a side face part 24 of the applying member. Reference numerals 26a and 26b shown in the drawing are a front end side face and a rear end side face for introducing the aqueous cosmetic into the passage 23 in the applying member 20.

The valve mechanism 16 is opened by pushing the above applying member 20, and the aqueous manicure composition H is introduced into the passage 23 in the applying member 20 and supplied to the applying tip 25 equipped with a slit in a conical and radial form.

In a use mode of the aqueous cosmetic-stored applicator of the present invention, when the applying member 20 of the applicator A shown in Fig. 1 is pushed, the aqueous cosmetic H which is the acid dye base aqueous cosmetic or the organic pigment base aqueous cosmetic of the present invention is transported from the liquid storing part 11 to the applying member 20, and therefore lines and patterns are drawn directly on a nail or drawn on a coating film of a nail enamel comprising nitrocellulose, a resin, acetate esters and the like applied in advance on a nail. Then, a transparent nail enamel is applied thereon to finish coating, whereby drawing is completed.

Further, the aqueous cosmetic-stored applicator of the present invention is equipped with the liquid storing part 11 stored with the aqueous cosmetic H which is the acid dye base aqueous cosmetic or the organic pigment base aqueous cosmetic having the characteristics described above and the applying member 20, and the aqueous cosmetic H having the respective compositions described above is transported from the liquid storing part 11 to the applying member 20 by virtue of a capillary force. An applying member provided with a capillary force is employed as the applying member 20 described above, whereby provided is the aqueous cosmetic-stored applicator which is excellent in usability to make it possible to readily draw fine lines and patterns on a nail, a skin and the like and which is excellent in a finishing property.

Further, the applying member 20 provided with a capillary force assumes structure in which it is equipped with the tubular inner cylinder 21 and in which the applying tip 25 is equipped with a slit in a conical and radial form, whereby capable of being prepared is the aqueous cosmetic-stored applicator which is more excellent in usability to make it possible to readily draw finer lines and patterns on a nail and the like and which is very excellent in a finishing property. On the other hand, in addition to the structure of the applying member 20 provided with a capillary force in which it is equipped with the tubular inner cylinder 21 and in which the applying tip 25 is equipped with a slit in a conical and radial form, and in addition thereto, it may assume as well a structure in which plural opening parts for allowing the aqueous cosmetic described above to flow in are provided. Usually, only opening parts (introduction holes 23a, 23b in the preset embodiment) in an end at an opposite side to the applying tip described above are provided, and a part of a side face of the tubular inner cylinder can be opened by cutting a side face of the applying member to form opening parts. Employing the above constitution makes it possible to secure a passage in causing about clogging by titanium oxide and the acid dye or the organic pigment each described above and in addition thereto, makes it possible to allow the aqueous cosmetic described above to flow in without delay even when troubles such as a rise in a viscosity of the aqueous cosmetic having the respective compositions described above by vaporization of water and the like are brought about.

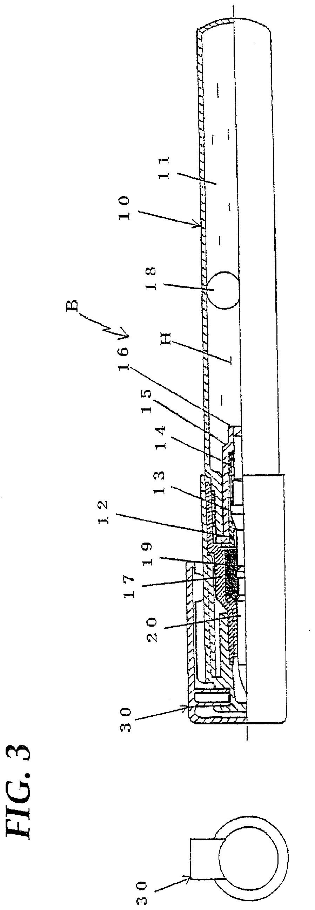

Also, in contrast to the aqueous cosmetic-stored applicator A of the embodiment described above, it may be, as shown in Fig. 3, an aqueous cosmetic-stored applicator B in which a porous member having continuous pores (for example, a sponge material) 19 is further provided in a passage of the aqueous cosmetic H extending from the liquid storing part 11 to the applying member 20.

In the aqueous cosmetic-stored applicator B of the present embodiment, the porous member having continuous pores (for example, a sponge material) 19 is provided in a rear of the applying member 20 and a periphery of the valve rod 13. Employing the aqueous cosmetic-stored applicator B of the above structure makes it possible to supply smoothly and efficiently the aqueous cosmetic having the characteristics described above to the applying member. For example, when an excess amount of the aqueous cosmetic flows out due to more opening of the valve mechanism 16 in Fig. 1 than necessary, it becomes possible to hold temporarily the excess amount thereof by the porous member having continuous pores 19 described above, and dripping of the aqueous cosmetic from the applying member 20 can be prevented.

### EXAMPLES

Next, the present invention shall be explained in further details with reference to examples and comparative examples, but the present invention shall not be restricted to the examples shown below.

### First invention, acid dye base aqueous cosmetics: Examples 1 to 8 and Comparative Examples 1 to 4

Blend components shown in the following Table 1 were mixed and dispersed by means of a homomixer or a disper to prepare aqueous manicure compositions which were the respective acid dye base aqueous cosmetics.

The respective aqueous manicure compositions obtained were used to evaluate a viscosity (initial) at a prescribed shear rate, a viscosity stability, a particle diameter (initial), an aggregation test, a hard cake test, followability and a masking power by the following evaluation methods and the like. The results thereof are shown in the following Table 1.

### Measuring method of viscosity, initial:

The respective aqueous cosmetics obtained were used to measure viscosities at the shear rates of 3.83 s⁻¹ and 383 s⁻¹ at a temperature of 25°C by means of a cone plate type viscometer (among TV-30 type viscometers, an ELD type viscometer or an EMD type viscometer, each equipped with a standard cone plate, manufactured by Tokimec Inc.).

### Measuring method of a viscosity stability:

The aqueous cosmetic was put in a tightly closed vessel and left standing under an environment of 50°C for one week, and after restored to 25°C, a viscosity thereof was measured by the same method as described above and evaluated according to the following evaluation criteria.

### Evaluation criteria:

○: difference is scarcely present between an initial viscosity value and a viscosity value after left standing at 50°C
Δ: a viscosity value after left standing at 50°C is risen a little as compared with an initial viscosity value
×: a viscosity value after left standing at 50°C is risen to a large extent as compared with an initial viscosity value or gelation is brought about after left standing at 50°C

### Measuring method of a particle diameter (initial), an aggregation test:

The respective aqueous cosmetics obtained were used to measure a particle diameter (nm) at 25°C by means of N4 Plus (manufactured by Beckman Coulter, Inc.).

### Aggregation test:

The aqueous cosmetic was put in a tightly closed vessel and left standing under an environment of 50°C for one week, and after restored to 25°C, a particle diameter thereof was measured by the same method as described above and evaluated according to the following criteria.

### Evaluation criteria:

○: difference is scarcely present between an initial particle diameter and a particle diameter after left standing at 50°C
Δ: a particle diameter after left standing at 50°C is increased a little as compared with an initial particle diameter
×: a particle diameter after left standing at 50°C is increased to a large extent as compared with an initial particle diameter or gelation is brought about after left standing at 50°C

### Evaluation method of followability:

The cosmetic loaded in a pen type vessel (storing applicator) was discharged and used, and then a discharge state of the cosmetic was evaluated according to the following criteria.

### Evaluation criteria:

○: the cosmetic can be applied smoothly
Δ: the cosmetic is skipped in some portions
×: the cosmetic is not discharged at all

### Hard cake test:

The cosmetic was loaded in a pen type vessel (storing applicator) and centrifugally separated at 1000 rpm for 30 minutes by means of a centrifugal separator H-40F (manufactured by KOKUSAN Co., Ltd.), and this was shaken up and down to count a frequency observed until the stirring ball moved and evaluate it according to the following criteria.

### Evaluation criteria:

○: 0 to less than 100
Δ: 100 to less than 200
×: 200 or more

### Evaluation method of a masking power:

The cosmetic was applied on a nail by means of the applying member, and then a state of masking a color of the base was evaluated according to the following criteria.

### Evaluation criteria:

O: the base is masked, and a color of the cosmetic is developed well
Δ: a color of the base is slightly observed, but a color of the cosmetic is developed well
×: a color of the base is observed, and a color of the cosmetic is developed badly

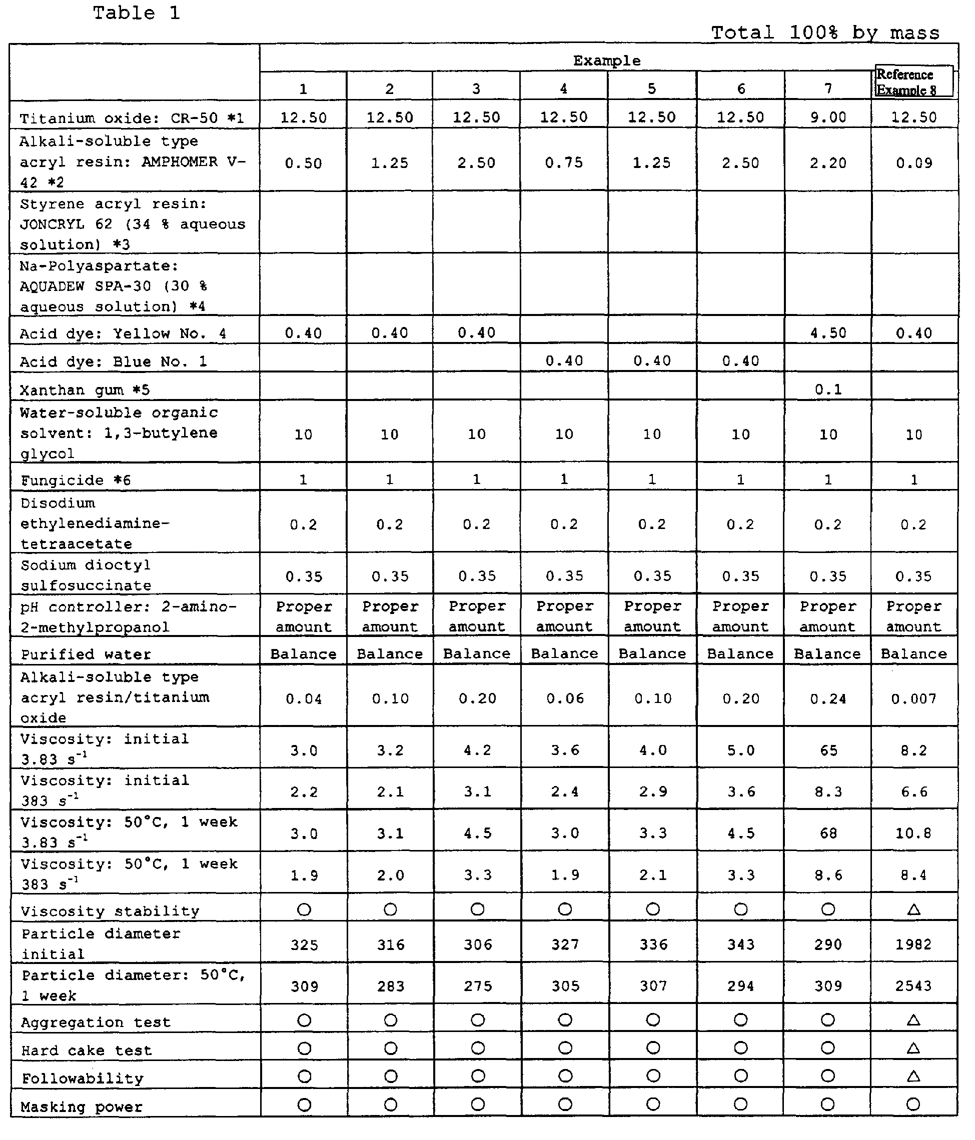

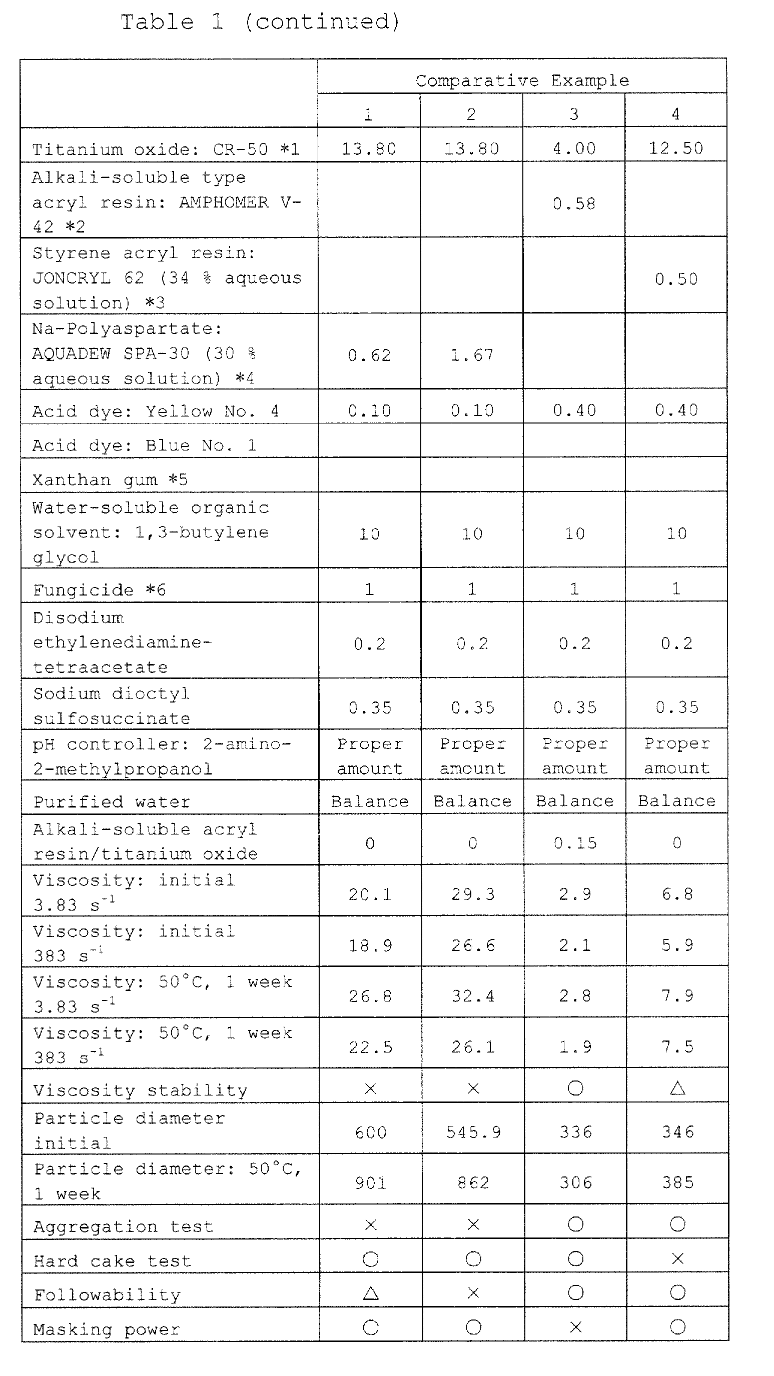

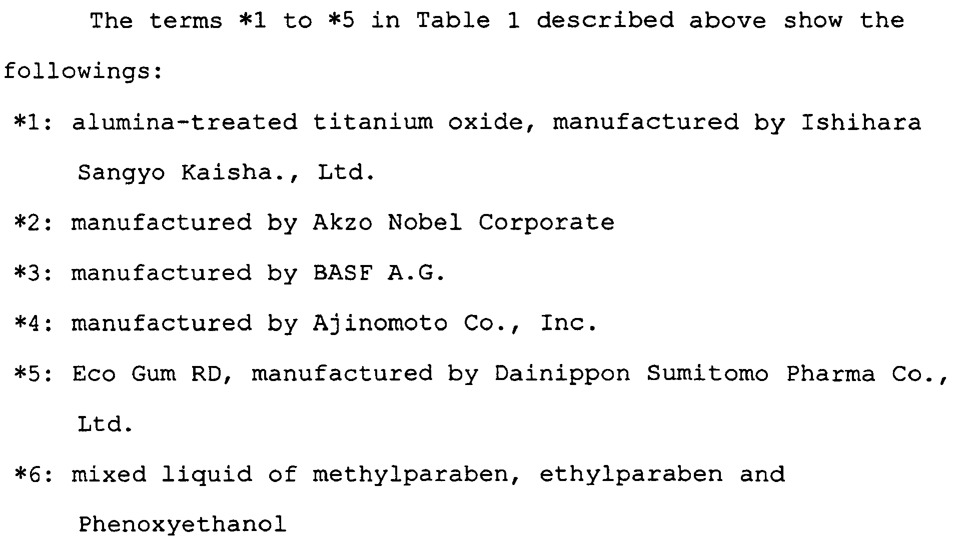

As apparent from the results shown in Table 1 described above, it has been found clear that the acid dye base aqueous cosmetics of the present first invention prepared in Examples 1 to 7 and Reference Example 8 are excellent in a viscosity and a stability of a particle diameter, do not causing about aggregation and are excellent in an aging settling stability and that in addition thereto, they mask the base and develop good colors as compared with those prepared in Comparative Examples 1 to 4 falling outside the scope of the present first invention.

To observe individually Comparative Examples 1 to 4, the alkali-soluble type acryl resin is not contained in Comparative Examples 1 and 2, and therefore it has been found clear that the aging stability is inferior and that a rise in the viscosity and an increase in the particle diameter are brought about. It has been found clear that in Comparative Example 3, a problem is not involved in the aging stability, but the masking power is weak due to a small content of titanium oxide. In Comparative Example 4, it has been found clear that the viscosity stability is inferior since the styrene-acryl resin is used and that a hard cake is produced.

### Second invention, organic pigment base aqueous cosmetics: Examples 9 to 18 and Comparative Examples 5 to 8

Blend components shown in the following Table 2 were mixed and dispersed by means of a homomixer or a disper to prepare aqueous manicure compositions which were the respective organic pigment base aqueous cosmetics.

The respective aqueous manicure compositions obtained were used to evaluate a viscosity (initial) at a prescribed shear rate, a hard cake test, followability and a masking power by the evaluation methods described above. The results thereof are shown in the following Table 2.

The viscosity stability, the aggregation test and the like which were evaluated in the acid dye base aqueous cosmetics described above were not evaluated in the examples in which the above organic pigment base aqueous cosmetics were prepared because of the following reasons; considering that titanium oxide is liable to be aggregated in a case in which the acid dye is added as compared with a case in which the organic pigment is added and that when sodium-polyaspartate is used as a dispersant for titanium oxide, the cosmetics are liable to be aggregated with the passage of time, the viscosity stability, the aggregation test and the like described above were further added and evaluated; and in a case of the organic pigment base aqueous cosmetics, it was considered that the effects of the organic pigment base aqueous cosmetics of the second invention could be confirmed and verified by the respective evaluations of the hard cake test, the followability and the masking power.

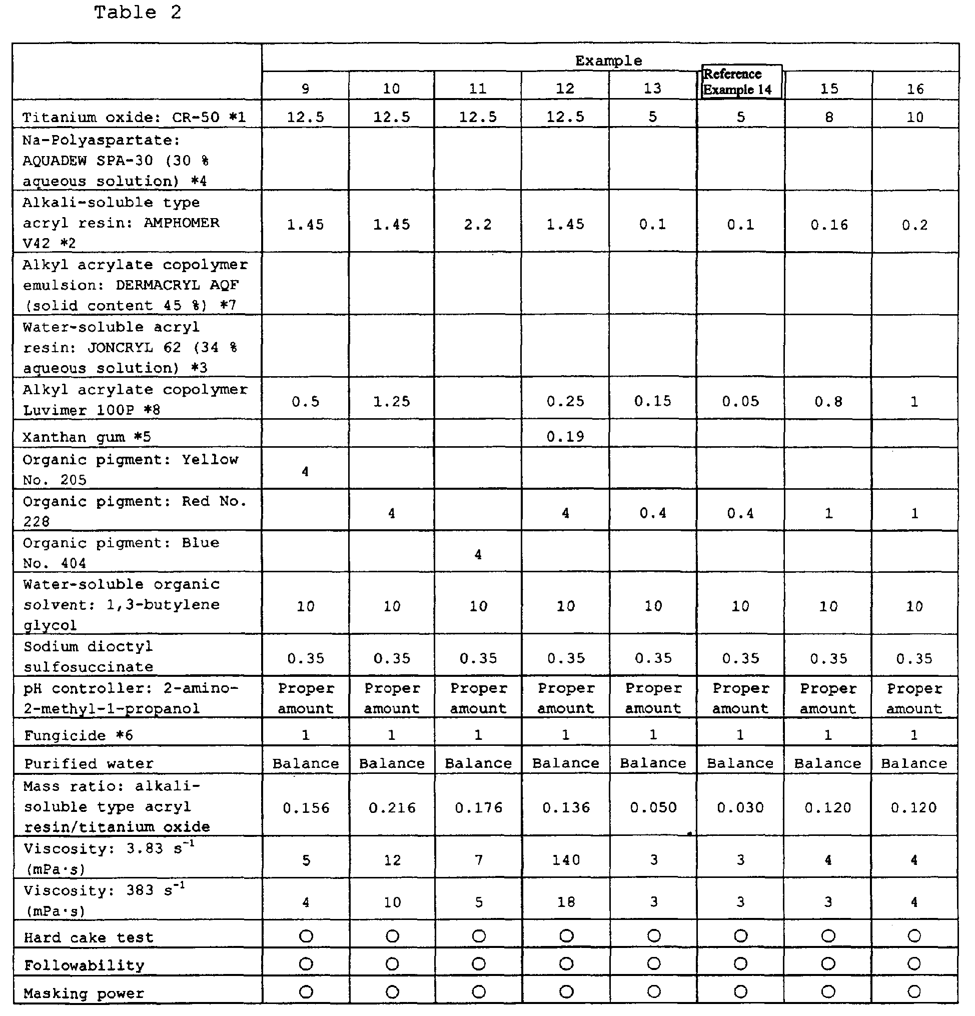

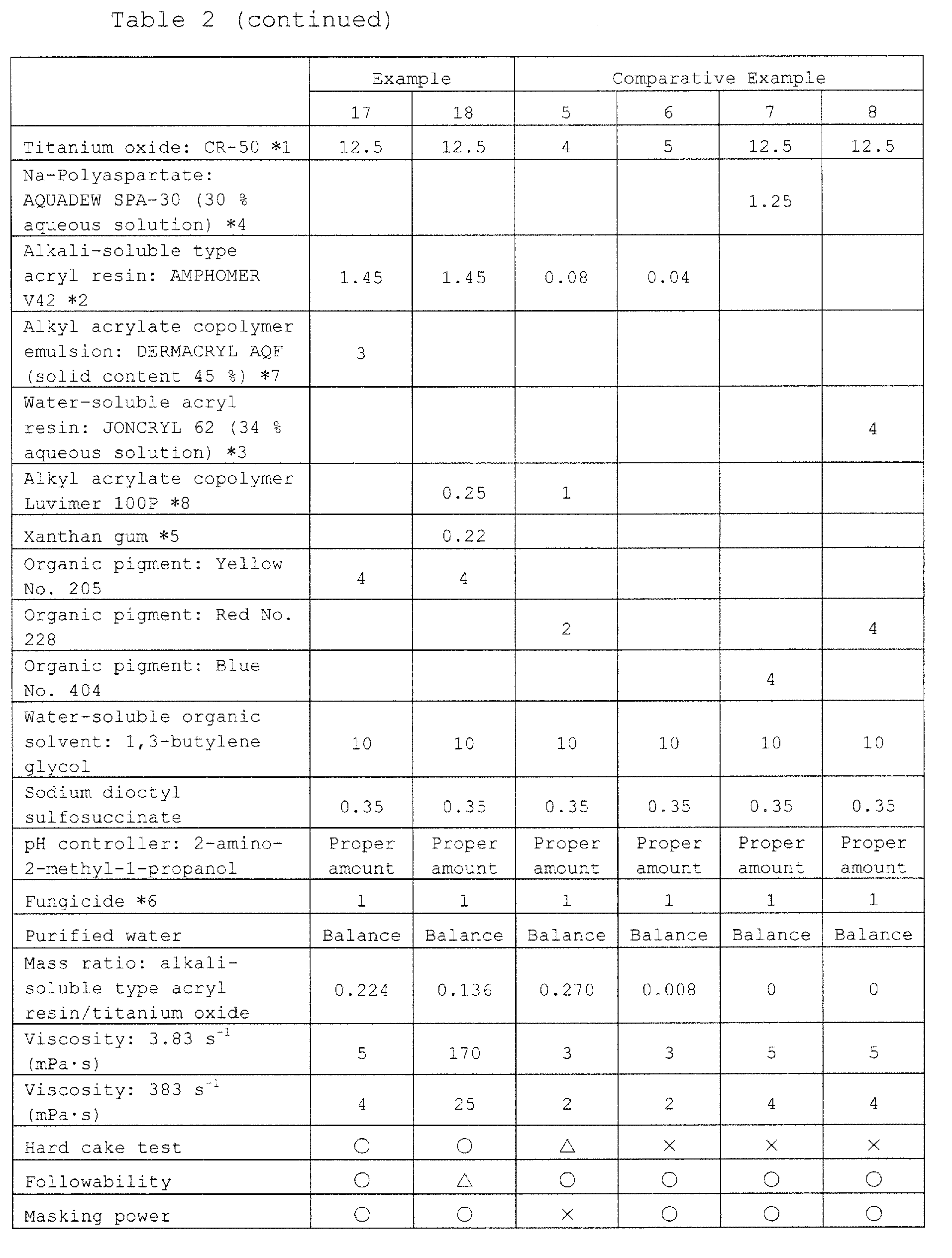

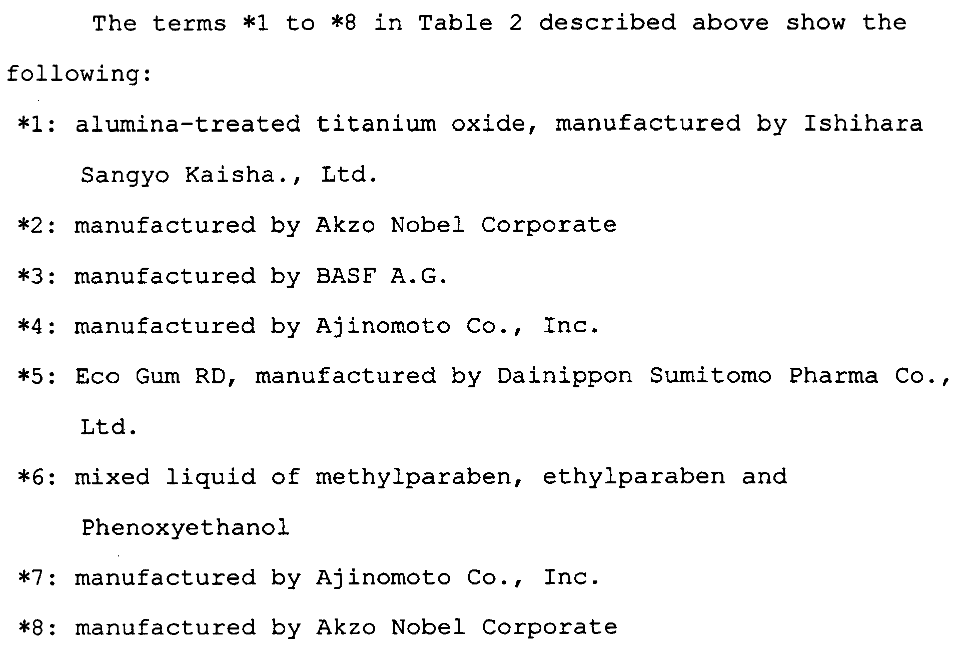

As apparent from the results shown in Table 2 described above, it has been found clear that from the test results of the hard cake, the organic pigment base aqueous cosmetics of the present second invention prepared in Examples 9 to 13, 15 to 18 and Reference Example 14 are excellent in an aging settling stability of titanium oxide to make a hard cake less liable to be produced even when titanium oxide settles down and can readily be redispersed by stirring and that the cosmetics can be applied smoothly without causing about starving to mask the base and develop good colors as compared with those prepared in Comparative Examples 5 to 8 falling outside the scope of the present invention.

To observe individually Comparative Examples 5 to 8, since a content of titanium oxide is small in Comparative Example 5, the masking power is inferior, and the alkali-soluble type acryl resin to titanium oxide is short in Comparative Example 6 to produce a hard cake of titanium oxide. It has been found clear that since the alkali-soluble type acryl resin is not contained in Comparative Examples 7 and 8, titanium oxide settles down to produce a hard cake and that it is not readily redispersed.

### Industrial Applicability

The aqueous cosmetic suited to manicure compositions for a nail art, cosmetics for skin coloring and the like is obtained.

### LETTERS and NUMERALS

- A: Aqueous cosmetic-stored applicator
- H: Aqueous cosmetic (aqueous manicure composition)
- 10: Applicator main body
- 11: Liquid storing part
- 16: Valve mechanism
- 17: Front shaft member
- 18: Stirring ball
- 20: Applying member
- 25: Applying tip (applying part)
- 30: Cap member

## Claims

1. An aqueous cosmetic containing at least 5 to 30 % by mass of titanium oxide, an acid dye, an alkali-soluble type acryl resin and water or containing at least 5 to 30 % by mass of titanium oxide, an organic pigment, an alkali-soluble type acryl resin and water, wherein the alkali-soluble type acryl resin is an (alkyl acrylate/octylacrylamide) copolymer, and wherein a mass ratio of the alkali-soluble type acryl resin to titanium oxide is 0.04 to 1, based on 1 of titanium oxide.

2. The aqueous cosmetic as described in claim 1, wherein titanium oxide coated on a surface with alumina is contained.

3. The aqueous cosmetic as described in any one of claims 1 to 2, wherein in measuring a viscosity of the aqueous cosmetic at 25°C by means of a cone plate type viscometer, a viscosity thereof at a shear rate of 3.83 s⁻¹ is 150 mPa·s or less, and a viscosity thereof at a shear rate of 383 s⁻¹ is 20 mPa·s or less.

4. The aqueous cosmetic as described in any one of claims 1 to 3, wherein the aqueous cosmetic is loaded in a liquid storing part of an applicator which is equipped with at least the liquid storing part and an applying member and in which a liquid is transported from the liquid storing part to the applying member by virtue of a capillary force.

## Patentansprüche

1. Eine wässrige Kosmetik, enthaltend mindestens 5 bis 30 Massen-% Titanoxid, einen Säurefarbstoff, ein Acrylharz vom alkalilöslichen Typ und Wasser oder enthaltend mindestens 5 bis 30 Massen-% Titanoxid, ein organisches Pigment, ein Acrylharz vom alkalilöslichen Typ und Wasser, wobei das Acrylharz vom alkalilöslichen Typ ein (Alkylacrylat/Octylacrylamid)-Copolymer ist, und wobei ein Massenverhältnis des Acrylharzes vom alkalilöslichen Typ zu Titanoxid 0,04 bis 1, bezogen auf 1 von Titanoxid, beträgt.

2. Die wässrige Kosmetik wie in Anspruch 1 beschrieben, wobei Titanoxid, das auf einer Oberfläche mit Aluminiumoxid beschichtet ist, enthalten ist.

3. Die wässrige Kosmetik wie in einem der Ansprüche 1 bis 2 beschrieben, wobei beim Messen einer Viskosität der wässrigen Kosmetik bei 25°C mithilfe eines Viskosimeters vom Kegel-Platte-Typ, eine Viskosität davon bei einer Schergeschwindigkeit von 3,83 s⁻¹ 150 mPa·s oder weniger beträgt, und eine Viskosität davon bei einer Schergeschwindigkeit von 383 s⁻¹ 20 mPa·s oder weniger beträgt.

4. Die wässrige Kosmetik wie in einem der Ansprüche 1 bis 3 beschrieben, wobei die wässrige Kosmetik in einem Flüssigkeitsspeicherteil eines Applikators geladen ist, der mit mindestens dem Flüssigkeitsspeicherteil und einem Auftragselement ausgestattet ist, und in dem eine Flüssigkeit von dem Flüssigkeitsspeicherteil zu dem Auftragselement durch eine Kapillarkraft transportiert wird.

## Revendications

1. Produit cosmétique aqueux contenant au moins 5 à 30 % en masse d'oxyde de titane, un colorant acide, une résine acrylique du type soluble dans les alcalis et de l'eau, ou contenant au moins 5 à 30 % en masse d'oxyde de titane, un pigment organique, une résine acrylique de type soluble dans les alcalis et de l'eau, dans lequel la résine acrylique du type soluble dans les alcalis est un copolymère d'acrylate d'alkyle/ octylacrylamide, et dans lequel le rapport en masse de la résine acrylique de type soluble dans les alcalis à l'oxyde de titane est de 0,04 à 1, sur la base de 1 pour l'oxyde de titane.

2. Produit cosmétique aqueux selon la revendication 1, qui contient de l'oxyde de titane revêtu sur une surface avec de l'alumine.

3. Produit cosmétique aqueux selon l'une quelconque des revendications 1 et 2, dans lequel, lors de la mesure de la viscosité du produit cosmétique aqueux à 25°C au moyen d'un viscosimètre du type plan-cône, la viscosité de celui-ci à une vitesse de cisaillement de 3,83 s⁻¹ est de 150 mPa·s ou moins, et la viscosité de celui-ci à une vitesse de cisaillement de 383 s⁻¹ est de 20 mPa·s ou moins.

4. Produit cosmétique aqueux selon l'une quelconque des revendications 1 à 3, ledit produit cosmétique aqueux étant chargé dans une partie de stockage de liquide d'un applicateur qui est équipé au moins de la partie de stockage de liquide et d'un élément d'application et dans lequel un liquide est transporté depuis la partie de stockage de liquide vers l'élément d'application en vertu d'une force capillaire.
